(19) Europäisches Patentamt
European Patent Office
Office européen des brevets

(11) EP 4 051 270 B1

(12) **EUROPEAN PATENT SPECIFICATION**

(45) Date of publication and mention of the grant of the patent:
**10.06.2026 Bulletin 2026/24**

(21) Application number: **20882313.8**

(22) Date of filing: **20.10.2020**

(51) International Patent Classification (IPC):
***C07D 471/04*** *(2006.01)* ***C07D 471/14*** *(2006.01)*
***A61K 31/437*** *(2006.01)* ***A61P 31/12*** *(2006.01)*
***A61P 35/00*** *(2006.01)* ***A61P 37/00*** *(2006.01)*

(52) Cooperative Patent Classification (CPC):
**C07D 471/04**

(86) International application number:
**PCT/US2020/056515**

(87) International publication number:
**WO 2021/086689 (06.05.2021 Gazette 2021/18)**

# (54) 4-AMINO-IMIDAZOQUINOLINE COMPOUNDS AND USE THEREOF

4-AMINO-IMODAZOCHINOLINVERBINDUNGEN UND VERWENDUNGEN DAVON

COMPOSÉS DE 4-AMINO-IMIDAZOQUINOLINE ET UTILISATION DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2019 US 201962927354 P**

(43) Date of publication of application:
**07.09.2022 Bulletin 2022/36**

(73) Proprietor: **Chou, Yu-Cheng**
**Hsinchu City (TW)**

(72) Inventor: **Chou, Yu-Cheng**
**Hsinchu City (TW)**

(74) Representative: **Gill, Siân Victoria et al**
**Venner Shipley LLP**
**TIDE Bankside**
**8 Emerson Street**
**London SE1 9DU (GB)**

(56) References cited:
**WO-A1-2004/058759 WO-A1-2012/167081**
**WO-A1-2014/201245 WO-A1-2015/162075**
**WO-A1-2019/048036 WO-A1-2020/160054**
**WO-A1-2020/252015 WO-A2-02/46189**
**WO-A2-2006/028962 US-A1- 2015 299 194**
**US-A1- 2019 062 329**

- **CE SHI ET AL: "Discovery of imidazoquinolines with Toll-like receptor 7/8 independent cytokine induction", ACS MEDICINAL CHEMISTRY LETTERS, AMERICAN CHEMICAL SOCIETY, US, vol. 3, no. 6, 1 January 2012 (2012-01-01), pages 501 - 504, XP008158663, ISSN: 1948-5875, DOI: 10.1021/ML300079E**
- **IRA MUSMUCA ET AL: "Discovery of imidazoquinolines with Toll-like receptor 7/8 independent cytokine induction", JOURNAL OF CHEMICAL INFORMATION AND MODELING, vol. 49, no. 7, 27 July 2009 (2009-07-27), US, pages 1777 - 1786, XP055517419, ISSN: 1549-9596, DOI: 10.1021/ci900065a**
- **SMITH ALYSON J ET AL: "Evaluation of novel synthetic TLR7/8 agonists as vaccine adjuvants", VACCINE, ELSEVIER, AMSTERDAM, NL, vol. 34, no. 36, 9 July 2016 (2016-07-09), pages 4304 - 4312, XP029649671, ISSN: 0264-410X, DOI: 10.1016/J.VACCINE.2016.06.080**
- **DATABASE Pubchem Compound ANONYMOUS : "1-Hydroxy-2-methylimidazo[4,5-c]quinolin-4-amine", XP055930456, retrieved from NCBI Database accession no. CID 57853775**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 051 270 B1

## Description

### FIELD OF THE INVENTION

The present invention relates generally to Toll-like receptor (TLR) agonists, and more specifically to TLR7 and TLR8 dual agonists.

### BACKGROUND OF THE INVENTION

Toll-like receptors (TLRs) are pathogen recognition receptors which play a critical role in activating both innate and adaptive immunity. Several TLRs have been identified in humans and mice. Some TLRs are found located on the cell surfaces (e.g., TLR 1, 2, 4, 5 and 6), and some are found located in the endosomal compartments (e.g., TLR 3, 7, 8 and 9). TLRs are expressed on different immune cells, especially monocytes, dendritic cells (DCs) and macrophages. The activation of TLRs results in cytokine secretion (e.g., IFN-$\alpha$, TNF-$\alpha$ and IL-12) and increased phagocytosis by macrophages and cytolytic activity by natural killer (NK) cells. TLRs activation also results in enhanced antigen presentation, thus activating an adaptive immune response, including antigen-specific $CD8^+$ cytotoxic T lymphocytes.

Small molecule TLR 7 and/or TLR 8 agonists have been identified in US Patent Nos. 8728486, 9334268, 9884866 and US Patent publication No. 20190062329. There is still a need for developing TLR7 and/or TLR8 agonists, especially TLR 7/8 agonists with improved potency and/or cytokine profile.

CE SHI et al (ACS Medicinal Chemistry Letters, June 2012, 3(6):501-504) disclose discovery of imidazoquinolines with Toll-Like Receptor 7/8 independent cytokine induction.

IRA Musmuca et al (J. Chem. Inf. Model. 2009, 49, 1777-1786), disclose small-molecule interferon inducers. Toward the comprehension of the molecular determinants through ligand-based approaches.

SMITH ALYSON J et al (Vaccine. 2016 July 9; 34(36): 4304-4312) disclose evaluation of novel synthetic TLR7/8 agonists as vaccine adjuvants.

WO 2015/162075 A1 discloses 4-amino-imidazoquinoline compounds.

WO 2020/160054 A1 discloses imidazoquinoline amine derivatives, pharmaceutical composition, use thereof.

WO 2006/028962 A2 discloses 1-Alkoxy 1H-imidazo ring systems and methods.

WO 2012/167081 A1 discloses hydrazine 1H-imidazoquinoline-4-amines and conjugates made therefrom.

WO 2020/252015 A1discloses immunomodulator antibody drug conjugates and use thereof.

WO 2019/048036 A I discloses substituted imidazoquinolines.

WO 2004/058759 A1 discloses aryl / hetaryl substituted imidazoquinolines.

WO 2002/046189 A2 discloses aryl ether substituted imidazoquinolines.

WO 2014/201245 A1 discloses TLR-9 agonist with TLR-7 and/or TLR-8 agonist for treating tumors.

### SUMMARY OF THE INVENTION

In one aspect, the invention relates to a pharmaceutical composition, comprising: 1-(4-amino-2-pentyl-1H-imidazo[4,5-c]quinolin-1-yl)-2-methylpropan-2-ol or a pharmaceutically acceptable salt thereof; and a pharmaceutically acceptable carrier and/or an adjuvant.

In one embodiment, a pharmaceutical composition according to the invention is for use in treating a viral infection, cancer, an allergic disease, or a liver disease in a subject in need thereof

The invention also relates to a compound with the structure of

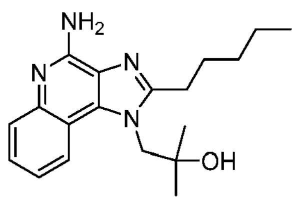

or a pharmaceutically acceptable salt thereof for use in treating a viral infection, cancer, an allergic disease or a liver disease in a subject in need thereof.

In one embodiment, cancer is selected from the group consisting of esophageal, stomach, colon, rectal, pancreatic, lung, breast, cervix uteri, corpus uteri, ovary, bladder, head and neck, endometrial, osteosarcoma, prostate and neuroblastoma.

Further in another aspect, the invention relates to a compound with the structure of

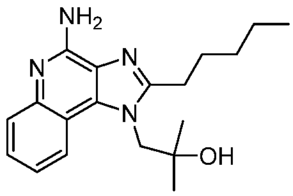

or a pharmaceutically acceptable salt thereof, or a pharmaceutical composition according to the invention, for use in treating a disease or a condition wherein activation of TLR7 and/or TLR8 provides a benefit in a subject in need thereof.

## BRIEF DESCRIPTION OF THE DRAWINGS

Figs. 1A-C depict the relative IL-6 induction from human PBMCs stimulated with various concentrations of test compounds (compounds **2, 9** and **10)** and reference compounds **(Ref. 1-3).** Compound **Ref. 1** is resiquimod (a TLR7/8 dual agonist). Compound **Ref. 2** is 1-(4-amino-2-butyl-1*H*-imidazo[4,5-c]quinolin-1-yl)-2-methylpropan-2-ol (a TLR7/8 dual agonist). Compound **Ref. 3** is VTX2337 (a TLR8 agonist).

Figs. 2A-C depict the relative IL-12p70 induction from human PBMCs stimulated with various concentrations of test compounds and reference compounds. Test compounds and reference compounds are the same as Figs. 1A-C.

Figs. 3A-C depict the relative IP-10 induction from human PBMCs stimulated with various concentrations of test compounds and reference compounds. Test compounds and reference compounds are the same as Figs. 1A-C.

## DETAILED DESCRIPTION OF THE INVENTION

### DEFINITIONS

The nomenclature used in this application is based on IUPAC systematic nomenclature, unless indicated otherwise.

As used herein, the article "a" or "an" refers to one or to more than one (i.e. to at least one) of the grammatical object of the article.

The term "pharmaceutically acceptable salts" refers to those salts which retain the biological effectiveness and properties of the free bases or free acids, which are not biologically or otherwise undesirable. Pharmaceutically acceptable salts include both acid and base addition salts. The salts are for example acid addition salts of compounds of formula (I) with physiologically compatible mineral acids, such as hydrochloric acid, hydrobromic acid, nitric acid, carbonic acid, sulfuric acid, sulfurous acid or phosphoric acid; or with organic acids, such as methanesulfonic acid, ethanesulfonic acid, p-toluenesulfonic acid, formic acid, acetic acid, propionic acid, glycolic acid, pyruvic acid, oxalic acid, lactic acid, trifluoroacetic acid, citric acid, fumaric acid, maleic acid, malonic acid, tartaric acid, benzoic acid, cinnamic acid, mandelic acid, embonic acid, succinic acid or salicylic acid. In addition, pharmaceutically acceptable salts may be prepared from addition of an inorganic base or an organic base to the free acid. Salts derived from an inorganic base include, but are not limited to, the sodium, potassium, lithium, ammonium, calcium, magnesium, zinc, copper, manganese and aluminium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, histidine, caffeine, procaine, hydrabamine, choline, betaine, ethylendiamine, glucosamine, methylglucamine, theobromine, piperazine, N-ethylpiperidine, piperidine and polyamine resins. The compound of formula (I) can also be present in the form of zwitterions. Pharmaceutically acceptable salts of compounds of formula (I) of particular interest are the sodium salts or salts with tertiary amines.

The term "half maximal effective concentration ($EC_{50}$)" means the plasma concentration of a particular compound required for obtaining 50% of the maximum of a particular effect in vivo.

The term "therapeutically effective amount" means an amount of a compound of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, disease state being treated, the severity or the disease treated, the age and relative health of the subject, the route and form of administration, the judgment of the attending medical or veterinary practitioner,

and other factors.

The term "treatment" or "treating" refers to the application or administration of a therapeutic agent, i.e. a compound provided herein, to a patient, or application or administration of a therapeutic agent to an isolated tissue or cell line from a patient (e.g., for diagnosis or ex vivo applications), who has a disease, a symptom of the disease or the potential to develop the disease, with the purpose to heal, alleviate, relieve, alter, remedy, ameliorate, improve or affect the disease, the symptoms of the disease, or the potential to develop the disease. Such treatments may be specifically tailored or modified, based on knowledge obtained from the field of pharmacogenomics.

The compounds of formula (I) and their pharmaceutically acceptable salts may be used as a medicament, in a form of a pharmaceutical composition/formulation suitable for enteral, parenteral or topical route of administration. The medicament may be used for systemic (e.g., parenteral) or local (e.g., topical or intralesional injection) administration. The routes of administration of the medicament may be topically, parenterally, vaginally, intrauterine, intranasal, or by inhalation.

Techniques for preparing various dosage forms of parenteral, oral, transdermal, and pulmonary are all well-known in the skill of the art. Effective doses will vary, as recognized by those skilled in the art, depending on route of administration, excipient usage, and the possibility of co-usage with other therapeutic treatment.

The "Guidance for Industry and Reviewers Estimating the Safe Starting Dose in Clinical Trials for Therapeutics in Adult Healthy Volunteers" published by the U.S. Department of Health and Human Services Food and Drug Administration discloses "a human equivalent dose" may be obtained by calculations from the following formula:

$$\text{HED} = \text{animal dose in mg/kg} \times (\text{animal weight in kg/human weight in kg})^{0.33}.$$

**Preparation of the compounds of the invention**

4-Amino-imidazoquinoline compounds disclosed below were synthesized and/or tested. Compound No. 2 relates to the subject of the invention. Compound Nos. 1, 9 and 10 in Table 1 and Compound Nos. 1 and 3-29 in Table 2 do not relate to the subject of the invention of the present application.

***General Synthesis Scheme***

Compounds of formula (I) may be made according to a general synthesis scheme shown below.

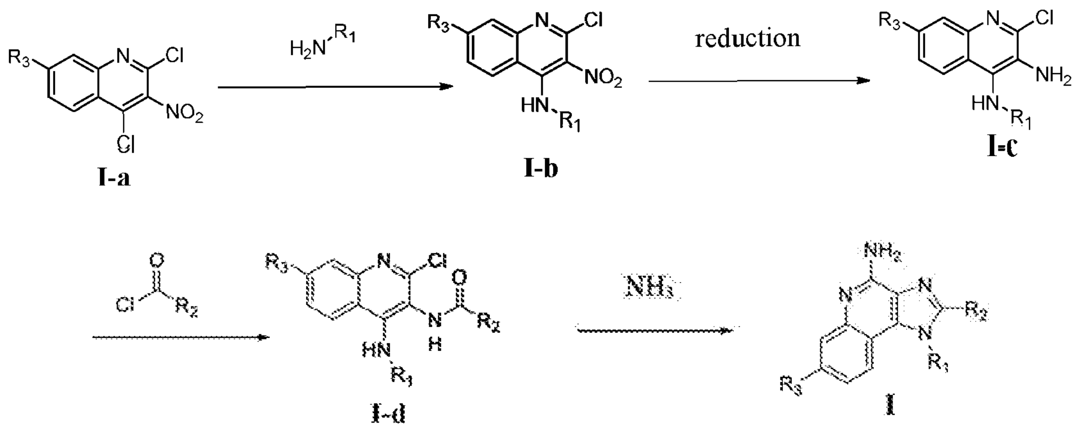

I-a
I-b
I-c
I-d
I

In general, a method of making a compound of formula (I comprises the steps of

(a) reacting a compound of formula (I-a)

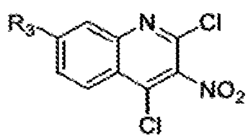
(I-a)

with $NH_2$-$R_1$ in the presence of a catalyst to obtain a compound of formula (I-b)

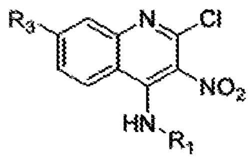

(I-b),

wherein $R_1$ and $R_3$ are as defined above;

(b) reducmg the compound of formula (I-b) in the presence of a reducing agent to obtain a compound of formula (I-c);

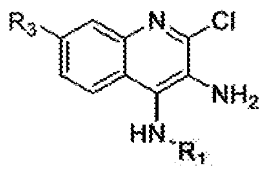

(I-c),

wherein $R_1$ and $R_3$ are as defined above,

(c) reaching the compound of formula (I-c) with Cl-C(O)-$R_2$ to obtain a compound of formula (I-d)

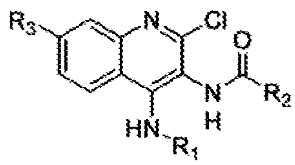

(I-d),

wherein $R_1$, $R_2$ and $R_3$ are as defined above; and

(d) reacting the compound of formula (I-d) with an ammoma solution to obtam the desired compound of formula (I)

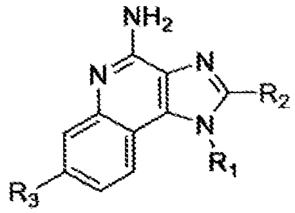

(I),

wherein $R_1$, $R_2$ and $R_3$ are as defined above

In step (a), the compound of formula (I-b) may be obtained in the presence of a solution of triethylamine (TEA) in dichloromethane (DCM) at a temperature between 40°C to 60°C preferably between 50°C to 60°C, or at 50°C for at least 1 hour, preferably for 1 to 4 hours, more preferably for 2 to 3 hours

In step (b), the compound of formula (I-b) maybe reduced in the presence of Fe/$NH_4Cl$ at a temperature between 70 C to 100°C preferably between 80°C to 90°C. or at 80°C for at least 1 hour, preferably for 1 to 4 hours, more preferably for 2 to 3 hours.

In step (c), the compound of formula (I-c) maybe dissolved in acetomtnle (ACN), and then reacts with Cl-C(O)-$R_2$ at a temperatute between 15°C to 35°C, or at a room temperature for at least 1 hour, preferably for 1 to 5 hours, more preferably for 2 to 4 hours

In step (d), the desired compound of formula (I) may be obtained in the presence of a solution of $NH_3$ in methanol at a temperature between 120°C to 180°C, preferably between 130°C to 160°C, or at150°C for at least 24 hours, preferably for 30 to 50 hours, more preferably for 35 to 40 hows

Compounds of Formula (I) may be prepared using the above general scheme Table 2 lists compounds of Formula (I). Synthesis of representive compounds are described in more detail below for illustration purpose.

***Synthesis of infermediate compound A***

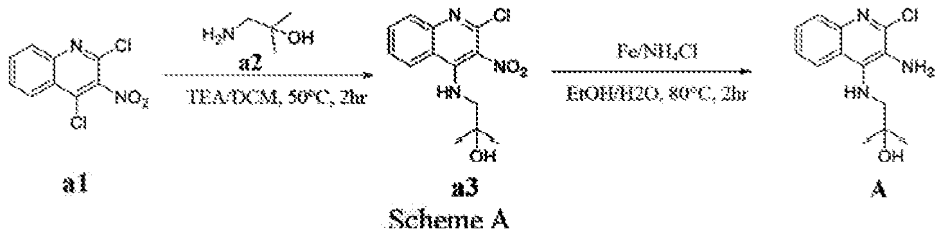


a1 a3 A

Scheme A

Compound **a1** was dissolved in dichloromethane (DCM) containing triethylamine (TEA) and was then selectively displaced by primary amine compound **a2** at 50°C for 2 hours to give nitroquinoline compound **a3** Then compound **a3** was mixed with EtOH/$H_2O$ and reduced by reacting with Fe/$NH_4Cl$ at 80°C for 2 hours to yield intermediate compound A (Scheme A) The resulting mtermediate compound **A** was used in the following examples.

***Analysis of test compounds***

All reactions were monitored for completion by thin layer chromatography (TLC) using Merck 60 $F_{254}$ silica gel glass backed plates (20 × 20 cm). Visualization of the resulting chromatograms was detected visually under UV irradiation (254 nm)

To analyze test compounds $^1$H NMR spectra were recorded on Varian Mercury-400 spectrometers and the chemical shifts were recorded in parts per million (ppm $\delta$). Multiplicities are recorded as s (singlet), br s (broad singlet) d (doublet), t (triplet), q (quartet) quin (quintet) sxt (sextet), and m (multiplet) Coupling constants (*J*) are expressed in hertz For liquid chromatography-mass spectrometry (LC-MS) electrospray mass spectra (ESMS) were recorded as *m*/*z* values using Waters mass spectrometer high-perfoumance liquid chromatography (HPI C) purity of the final compound was determined with Waters ACQUITY Arc system using C18 column (Waters XSelect HSS T3 column, 5 μm, 4 6 mm x 250 mm) operating at 40 °C Elution was carried out using water containing 0.1% trifluoroacetic acid and methanol as mobile phases The flow-rate of the mobile phase was 1 mL min Peaks were detec ed at 210-400 nm.

***Synthesis of Compound No. 1***

1-(4-ammo-2-propyl-1*H*-imidazo[4,5-c]quinolin-1-yl)-2-methylpropan-2-ol (compound **1**) was synthesized according to scheme 1.

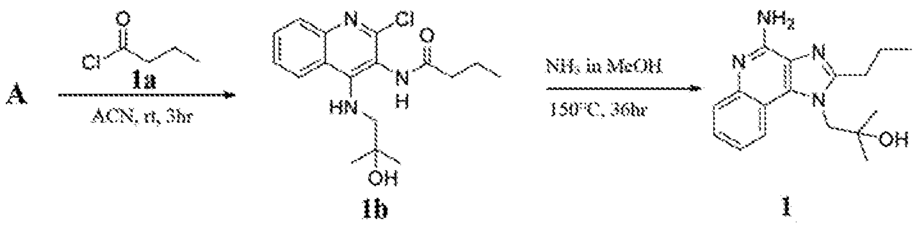


A 1b 1

Scheme 1

Intermediate compound **A** was dissolved in acetonitrile (ACN) and reacted with butyryl chloride (compound No **1a**) at room temperature for 3 hours to yield corresponding amme compound No **1b.** Compound No **1b** was reacted with ammonia solution (7N in methanol) at 150 °C for 36 hours to result in formation of an imidazole ring and displacement of the 4-chloro substituent to yield compound No 1. The crude product was purified by column chromatography to give the title compound as a beige solid $^1$H NMR (400 MHz, DMSO-*d6*) δ 8 26 (d, *J* = 8.0 Hz, 1 H) 7 57 (d, *J* = 8.0 Hz, 1 H), 7.36 (t *J* = 8.0 Hz, 1 H), 7.18 (t *J* = 8.0 Hz, 1 H), 6.40 (s, 2 H), 4.78 (s, 1 H) 4.58 (br s, 2 H) 3.00 (t, *J* = 7.5 Hz, 2 H), 1.82 (sxt, *J* = 7.4 Hz, 2 H), 1.17 (s, 6 H), 1.00 (t, *J* = 7.4 Hz, 3 H) LCMS (ESI) m/z 299.3 $[M+H]^+$. HPLC Purity: 99.65%.

***Synthesis of Compound No. 2***

1-(4-amino-2-pentyl-1*H*-imidazo[4,5-c]quinolin-1-yl)-2-methylpropan 2 ol (compound 2) was synthesized according to scheme 2

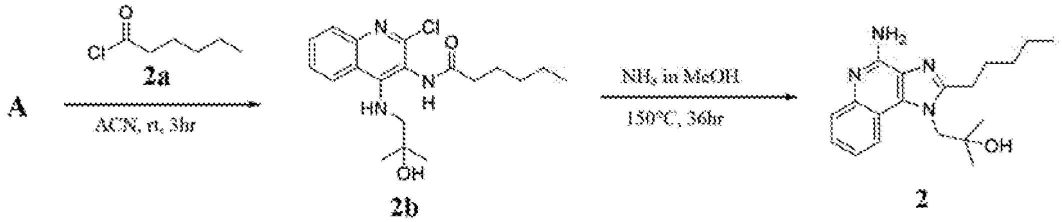


2b 2

Scheme 2

Intermediate compound **A** was dissolved in acetonitrile (ACN) and then reacted with hexanoyl chlonde (compound No **2a)** at room temperature for 3 hours to yield corresponding amine compound No **2b** Compound No **2b** was reacted with ammonia solution (7N in methanol) at 150 °C for 36 hours to result in formation of an imidazole ring and displacement of 4-chloro substituent to yield compound No **2** The crude product was punfied by column chromatography to give the title compound as a beige powder. $^1$H NMR (400 MHz, DMSO-*d6*) δ 8 25 (d *J* = 8 Hz, 1 H), 7.57 (d *J* = 7.8 Hz 1 H) 7.36 (t *J* = 7.8 Hz, 1 H), 7 18 (t, *J* = 7.8 Hz, 1 H) 6.40 (s, 2 H), 4 78 (s, 2 H), 4.54 (br s, 2 H), 3.01 (t, *J* = 7.8 Hz, 2 H) 1.80 (quin, *J* = 7.2 Hz, 2 H), 1.49-1.28 (m, 4 H) 1.17 (s., 6 H), 0.89 (t *J* = 7.2 Hz, 3 H) LCMS (ESI) *m/z* 327.4 $[M+H]^+$. HPLC Purity: 99.42%.

***Synthesis of Compound No. 9***

1-(4-amino-2-(4,4 4-trifluorobutyl)-1*H*-imidazo[4,5-c]quinolin-1-yl)-2-methylpropan-2-ol (compound No **9)** was synthesized according to scheme 4

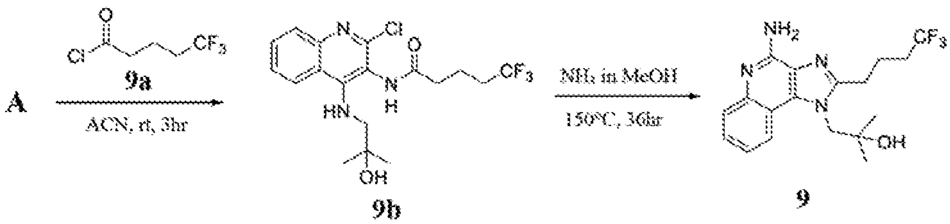


9b 9

Scheme 4

Intermediate compound **A** was dissolved in acetonitrile (ACN) and reacted with 5,5,5-tnfluoropentanoyl chloride (compound No **9a)** at room temperature for 3 hours to yield corresponding amine compound No **9b** Compound No **9b** was reacted with ammonia solution (7N in methanol) at 150 °C for 36 hours to result in for mation of an imidazole ring and displacement of the 4-chloro substituent to yield compound No **9**. The crude product was purified by column chromatography to give the title compound as a white powder. $^1$H NMR (400 MHz, DMSO-*d6*) δ 8.26(d *J* = 7.8 Hz, 1H), 7.58 (d, *J* = 7.8 Hz, 1 H) 7.37 (t, *J* = 7.8 Hz, 1 H), 7.19 (t, *J* =*7.8* Hz, 1 H), 6.44 (s, 2 H) 4.80 (s 1 H) 4.55 (br s 2 11), 3 12 (t *J* = 7.5 Hz, 2 H) 2.48-2.34 (m, 2 H) 206 (quin *J* = 7.5 Hz, 2 H), 1.17 (s, 6 H) LCMS (ESI) m/z 367.4 $[M+H]^+$, HPLC Purity 96 95%.

***Synthesis of Compound No. 10***

1-(4-amino-2-(5,5,5-influoropentyl)-1*H*-imidazo[4,5-c]quinolin-1-yl)-2-methylpropan-2-ol (compound No **10**) was synthesized according to scheme 5.

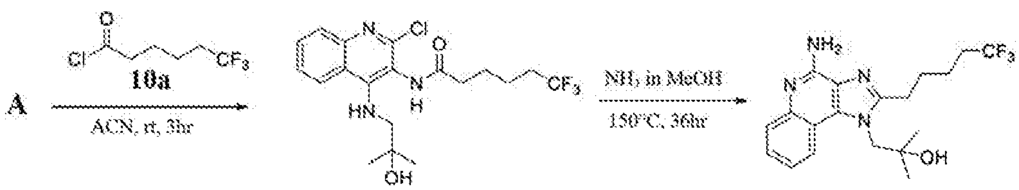


10b 10

Scheme 5

Intermediate compound **A** was dissolved in acetonitrile (ACN) and reacted with 6 6,6-tnfluorohexanoyl chloride (compound No **10a)** at a room temperature for 3 hours to yield corresponding amine compound No **10b** Compound No **10b**

was reacted with ammonia solution (7N in methanol) at 150 °C for 36 hours to result in formation of an imidazole ring and displacement of the 4-chloro substituent to yield Compound No **10** The crude product was purified by column chromatography to give the title compound as a white powder $^1$H NMR (600 MHz, DMSO-*d*6) δ 8.26 (d, *J* = 8 4 Hz, 1H), 7 57 (dd *J* = 8.4, 1.2 Hz, 1H), 7.36 (ddd, *J* = 8.4 7 0, 1.3 Hz, 1H), 7 20 (ddd *J* = 8.4, 7.0, 1.3 Hz 1H), 6.37 (s, 2H), 4.48 (s 1H), 4 55 (br s, 2H), 3.06 (t *J* = 7.8 Hz, 2H), 2.37-2.29 (m 2H), 1.90 (quintet, *J* = 7.8 Hz, 2H). 1.64 (quintet *J* = 7.8 Hz, 2H), 1.17 (br s 6H) I CMS (ESI) *m/z* 381.4 $[M +H]^+$. HPI C purity: 99.34%

Using simular synthesis schemes described above, other compounds listed in Table 2 could be readily generated For example, Compound No **3** was prepared using a procedure similar to the synthesis of compound 1 but using 1-methylbutanoyl chlonde instead of butyryl chlonde Compound No **22** was prepared using a procedure similar to the synthesis of Compound No 9 but using 3 ammo-2-chloro-4-((2-hydroxy-2-methvipropyl)amino)quinolin-7-ol instead of Intermediate compound **A.**

***In vitro TLR7 and TLR8 agonistic activity analysis***

To analyze the hTLR7 agonistic activity, HEK-Blue hTLR7 cells translected with secreted embryonic alkaline phosphatase (SEAP) reporter were seeded at $3\times10^4$ cell/well. The cells were treated with various concentrations of test compounds (Compound Nos. **1, 2, 9, 10**). Two reference compounds were used for comparisons **Ref. 1** (resiquimod, a TLR7 8 dual agonist) and **Ref. 2** (1-(4-amino-2-buty1-1*H*-imidazo[4,5-c]quinolin-1-yl)-2-methylpropan-2-ol **Ref 2** is a more potent TLR7/8 dual agonist than resiquimod See Ganapathi L et al (2015) PLOS ONE 10(8): e0134640) The SEAP reporter is designed specifically for measunng secreted placental AP (alkaline phosphatase) in conditioned cell culture medium from transfected cells The HEK Blue hTLR7 cells served to measure the bioactvity of TLR7 through SEAP upon NF-κB activation following TLR7 stimulation

To analyze the hTLR8 agonistic activity, HEK-Blue hTLR8 cells transfected with SE AP reporter were seeded at $4\times10^4$ cell/well The cells were treated with various concentiations of test compounds (Compound Nos **1, 2, 9, 10).** Two reference compounds **Ref. 1** and **Ref. 2** were used for comparisons The IIEK Blue hTLR8 cells served to measure the bioactivity) of TLR8 through the SEAP upon NF-κB activation following TLR8 stimulation.

In the SEAP reporter gene assay as described above water was used as a negative control After about 16 hours of incubation at 37 °C in 5% $CO_2$, the SE AP was deteimined by using a spectiophotometer at a wavelength of 635 nm. The dose-response curves were fit with four parameter logistic curves (SigmaPlot version 9.0). The $EC_{50}$-values of the test compounds and reference compounds were calculated Table 1 shows the results of the analysis of TLR7 and TLR8 agonistic activities.

Table 1

| Compound No | TLR7 $EC_{50}$ (nM) | TLR8 $EC_{50}$ (nM) | Ratio of TLR8 $EC_{50}$ and TLR7 $EC_{50}$ |
|---|---|---|---|
| **1** | 236.9 ± 7.4 | 5299.1 ± 14.4 | 22.36 |
| **2** | 67.1 ± 8.3 | 267.1 ± 13.5 | 3.98 |
| **9** | 128.8 ± 10.9 | 965.4 ± 14.7 | 7.49 |
| **10** | 542.8 ± 29.4 | 4745.6 ± 20.5 | 8.74 |
| **Ref. 1** | 161.9 ± 28.6 | 1698.2 ± 44.2 | 10.48 |
| **Ref. 2** | 24.0 ± 3.2 | 689.8 ± 27.5 | 28.74 |

Table 1 shows that all the test compounds exhibited TLR7 and TLR8 dual agonistic activities Among those, Compound No **2** was the most potent TLR718 dual agonist due to its low $EC_{50}$ of TLR7 and TI R8 agonistic activity. In addition, the ratios of TI R8 $EC_{50}$ and TLR7 $EC_{50}$ of the Compound Nos **2, 9, 10** were lower than the two reference compounds which indicates that these compounds could bring advantages of activating TLR7 and TLR8 at a very closed potency ratio and precisely modulating the activation of TLR7 and TLR8.

***Ex vivo cytokines profile analysis***

Three test compounds (Compound Nos **2, 9 10)** were used to evaluate the effect on cytokine induction. Three reference compounds were used for comparisons: **Ref. 1**(resiquimod, a TLR7/8 dual agonist), **Ref. 2** (1-(4-amino-2-butyl-1*H*-imidazo[4,5-c]quinolin-1-yl)-2-methylpropan-2-ol, a FLR7/8 dual agonist) and **Ref. 3** (Motolimod or VTX2337, a TLR8 agonist)

Cryopreserved human PBMCs fiom 4 noimal adult volunteers were purchased from EPBMC® (IMMUNOSPOT®) The PBMCs were thawed at 37°C for 10 minutes and centrifuged at 300×g at room temperature lor 5 minutes The pelleted PBMCs were transferred to RPMI-1640 medium containing 10% FBS, 1% PSA and incubated at 37°C with 5% CO2.

The test compounds and reference compounds were respectively dissolved in DMSO and subsequently 25-fold diluted with PBS to make stock solutions at a concentration of 0.1 mM The preincubated PBMCs were harvested and resuspended in a fresh RPMI-1640 medium containing 10% FBS and 1% PSA Approximately $1\times10^5$ PBMCs were added to each well of 96-well plates and stimulated with 0, 20 or 1000 nM of test compounds for 24 h at 37°C in a 5% $CO_2$ humidified incubator. The culture supernatant was stocked at -20°C for analysis of TLR7- and TLR8-mediated cytokine induction PROCARTAPLEX™ Immunoassay (Invitrogen) was performed to quantify human IL-6 IL-12p70, IP-10 in the culture supernatant according to the manufacturer's instructions. Briefly, the culture supernatant samples were thawed on ice and transferred to a microtiter plate. Magnetic bead coupled antibodies specific to the target cytokines were added to the samples for analyte capturing The immobilized target cytokine was specifically detected by biotin-conjugated antibodies and then labelled with streptavidin- Phycoerythrin (PE) The PE labelled magnetic beads were detected and identified by LUMINEX™ 200™ analyzer. Data acquisition, processing and analysis was conducted by using XPONENT™ 3.1 software The target cytokines were normalized to their baseline cytokine levels and presented as relative cytokines.

Figs 1A-1C show the relative IL-6 induction from human PBMCs stimulated with various concentrations of test compounds (Compound Nos **2 9** and **10)** and reference compounds **(Ref. 1-3)** Compound Nos **2 9** and **10** all showed low IL-6 induction activity, which are similar to **Ref 1** and **3** These results indicated that the compounds of the invention do not over induce IL-6 expression which is well known to play a pathological effect on chromc inflammation and autoimmumty due to dysregulated continual synthesis of IL-6. To the contrary, **Ref. 2** induced high IL-6 expression when treated at a concentration over 100 nM, particular over 1000 nM which meant that **Ref. 2** is not suitable for drug development due to a risk of over-inducing IL-6 expression Based on the structure sunilanty of the Compounds **2. 9 10** and **Ref. 2,** the low IL-6 induction activity of the compounds of the invention was unexpected

Figs 2A-2C show the relative IL-12p70 aduction from human PBMCs stimulated with various concentrations of test compounds (Compound Nos **2, 9** and **10**) and reference compounds (**Ref. 1-3**) Compound Nos **2 9** and **10** all showed high IL-12p70 induction activity, wherein the cytokine IL-12 is well known to mediate enhancement of the cytotoxic activity of NK cells and CD8 cytotoxic T cells and anti-angtogenic activity.

Figs. 3A-3C show the relative IP-10 Induction from human PBMCs stimulated with various concentrations of test compounds (Compound Nos **2, 9** and **10**) and reference compounds (**Ref**. **1-3**) Compound Nos **2, 9** and **10** all showed high IP-10 mduction activity, wherein cytokine IP-10 is well known to involve in inhibiting angiogenesis and is associated with anti-tumor activity.

Collectively the compounds of Formula (I) could simultaneously activate human TLR7 and TLR8, increase expression of 1L-12 and IP-10 without over-inducing undesired IL-6 expression

Table 2

| No. | Compound name | Structure |
|---|---|---|
| 1 | 1-(4-ammo-2-propyl-1*H*-imidazo[4,5-c]quinolin-1-yl)-2-methylpropan-2-ol | 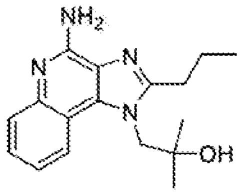 |
| 2 | 1-(4-ammo-2-pentyl-1*H*-imidazol[4,5-c]quinolin-1-yl)-2-methylpropan-2-ol | 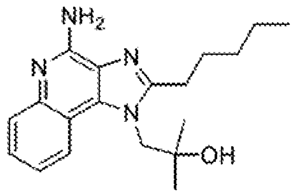 |
| 3 | 1-(4-amino-2-isobutyl-1*H*-imidazo[4,5-c]quinolin-1-yl)-2-methylpropan-2-ol | 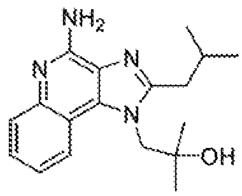 |

(continued)

| No. | Compound name | Structure |
|---|---|---|
| 4 | 1-(4-ammo-2-isopentyl-1*H*-imidazo[4,5-c]quinolin-1-yl)-2-methylpropan-2-ol | 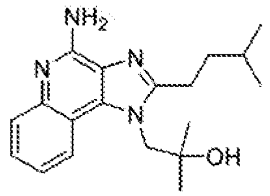 |
| 5 | 1-(4-amino-2-(4-methylpentyl)-1*H*-imidazo[4,5-c]quinolin-1-yl)-2-methylpropan-2-ol | 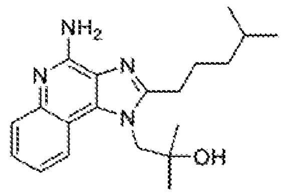 |
| 6 | 1-(4-amno-2-trifluoromethyl-1*H*-imidazo[4,5-c]quinolin-1-yl)-2-methylpropan-2-ol | 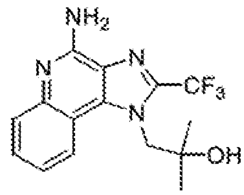 |
| 7 | 1-(4-ammo-2-(2.2,2-trifluoroethyl)-1*H*-imidazo[4,5-c]quinolin-1-yl)-2-methylpropan-2-ol | 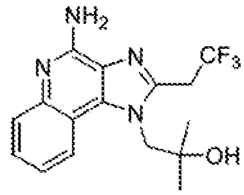 |
| 8 | 1-(4-amino-2-(3,3,3-trifluoropropyl)-1*H*-imidazo[4,5-c]quinolin-1-yl)-2-methyilpropan-2-ol | 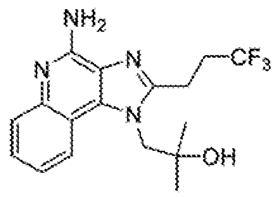 |
| 9 | 1-(4-amino-2-(4,4 4-trifluorobutyl)-1*H*-imidazo[4,5-c]quinolin-1-yl)-2-methylpropan-2-ol | 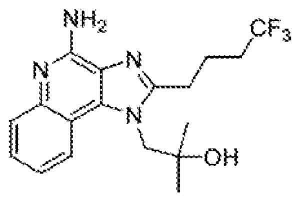 |
| 10 | 1-(4-amino-2-(5,5,5-trifluoropenty)-1*H*-imidazo[4,5-c]quinolin-1-yl)-2-methylpropan-2-ol | 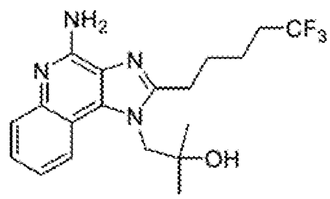 |
| 11 | 1-(4-amino-2-(6,6,6-trifluoroheryl)-1*H*-imidazo[4,5-c]quinolin-1-yl)-2-methylpropan-2-ol | 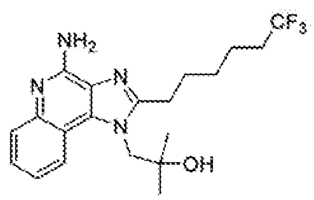 |

(continued)

| No. | Compound name | Structure |
|---|---|---|
| 12 | 1-(4-amino-2-(3,3,3-trifluoro-2-methylpropyl)-1*H*-imidazo[4,5-c]quinolin-1-yl)-2-methylpropan-2-ol | 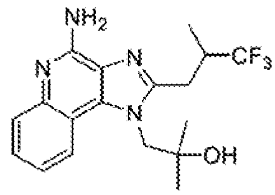 |
| 13 | 1-(4-amino-2-(4,4,4-trifluoro-3-methylbutyl)-1*H*-imidazo[4,5-c]quinolin-1-yl)-2-methylpropan-2-ol | 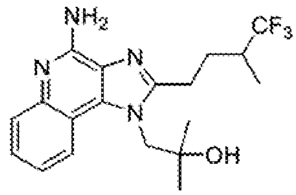 |
| 14 | 1-(4-amino-2-(5,5,5-trifluoro-4-methylpentyl)-1*H*-imidazo[4,5-c]quinolin-1-yl)-2-methylpropan-2-ol | 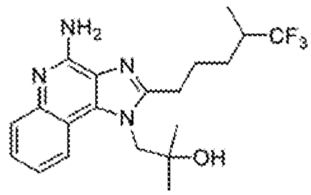 |
| 15 | 1-(4-amino-2-(2-(2,2,2-trifluoroethoxy)ethyl)-1*H*-imidazo[4,5-c]quinolin-1-yl)-2-methylpropan-2-ol | 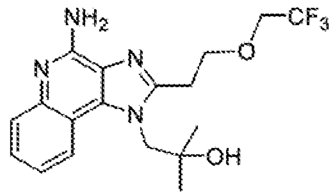 |
| 16 | 1-(4-amino-2-((3,3,3-tritluoropropoxy)methyl)-1*H*-imidazo[4.5-c]quinolin-1-yl)-2-methylpropan-2-ol | 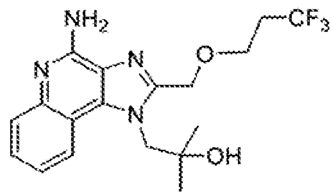 |
| 17 | 1-(4-ammo-2-(((2,2.2-trifluoroethynthto)methyl)-1*H*-imidazo[4,5-c]quinolin-1-yl)-2-methylpropan-2-ol | 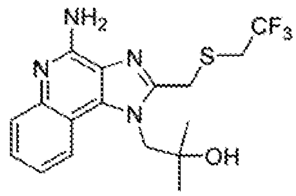 |
| 18 | 1-(4-amino-2-(((3,3,3-trifluoropropyl)thio)methyl)-1*H*-imidazo[4,5-c]quinolin-1-yl)-2-methylpropan-2-ol | 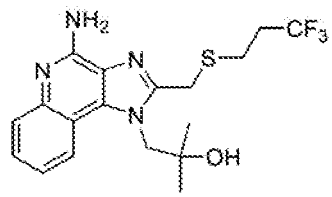 |
| 19 | 1-(4-amino-2-(((4,4,4-trifluorobutyl)thio)methyl)-1*H*-imidazo[4,5-c]qunolin-1-yl)-2-methylpropan-2-ol | 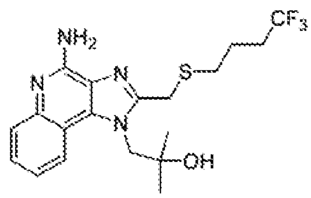 |

(continued)

| No. | Compound name | Structure |
|---|---|---|
| 20 | 1-(4-amino-2-(2-hydroxyethyl)-1*H*-imidazo[4,5-c]quinolin-1-yl)-2-methyl-propan-2-ol | 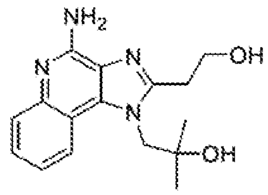 |
| 21 | 1-(4-ammo-2-(3-hydroxypropyl)-1*H*-imidazol 4,5-c]quinolin-1-yl)-2-methylpropan-2-ol | 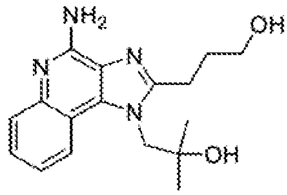 |
| 22 | 1-(4-amino-2-(4-hydroxybutyl)-1*H*-imidazo[4,5-c]quinolin-1-yl)-2-methyl-propan-2-ol | 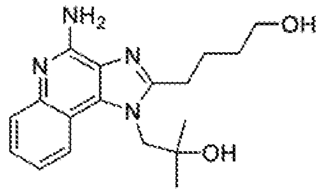 |
| 23 | 4-amino-2-butyl-1-(2-hydroxy-2-methylpiopyl)-1*H*-imidazo[4,5-c]quino-lin-7-ol | 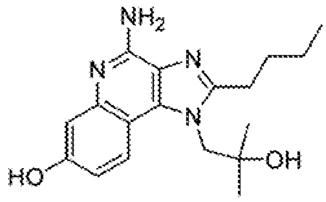 |
| 24 | 4-amino-2-(4,4,4-trifluorobutyl)-1-(2-hydroxy-2-methylpropyl)-1*H*-imidazo[4,5-c]quinolin-7-ol | 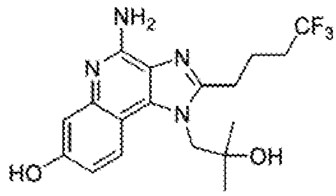 |
| 25 | 1-(4-amino-2-butyl-7-methoxy-1*H*-imidazo[4,5-c]quinolin-1-yl)-2-methyl-propan-2-ol | 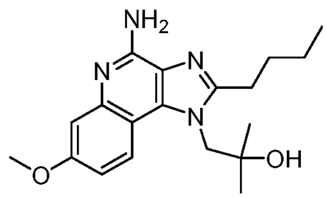 |
| 26 | 1-(4-amino-2-butyl-7-(hydroxymethyl)-1*H*-imidazo[4,5-c]quinoline-1-yl)-2-methylpropan-2-ol | 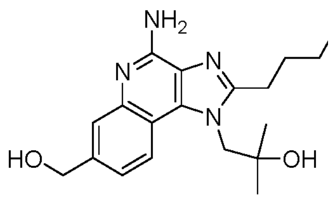 |

(continued)

| No. | Compound name | Structure |
|---|---|---|
| 27 | 1-(4-amino-2-butyl-7-fluoro-1*H*-imidazo[4,5-c]quinolin-1-yl)-2-methylpropan-2-ol | 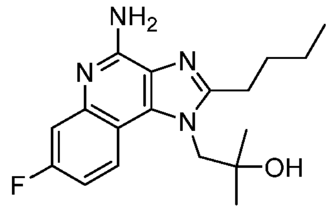 |
| 28 | 1-(4-amino-2-butyl-7-methyl-1*H*-imidazo[4,5-c]quinolin-1-yl)-2-methylpropan-2-ol | 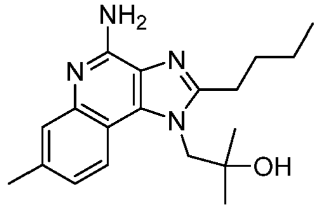 |
| 29 | 1-(4-amino-2-butyl-7-(trifluoromethyl)-1*H*-imidazo[4,5-c]quinolin-1-yl)-2-methylpropan-2-ol | 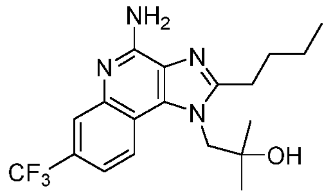 |

**Claims**

1. A pharmaceutical composition, comprising:

   1-(4-amino-2-pentyl-1*H*-imidazo[4,5-c]quinolin-1-yl)-2-methylpropan-2-ol or a pharmaceutically acceptable salt thereof ; and
   a pharmaceutically acceptable carrier and/or an adjuvant.

2. A pharmaceutical composition as claimed in claim 1 for use in treating a viral infection, cancer, an allergic disease, or a liver disease in a subject in need thereof.

3. A compound with the structure

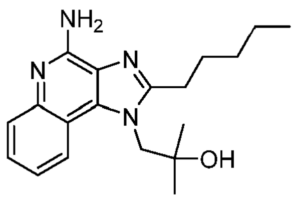

   or a pharmaceutically acceptable salt thereof for use in treating a viral infection, cancer, an allergic disease, or a liver disease in a subject in need thereof.

4. A compound or a pharmaceutically acceptable salt thereof as claimed in claim 3, or a pharmaceutical composition for use as claimed in claim 2, wherein the cancer is selected from the group consisting of esophageal, stomach, colon, rectal, pancreatic, lung, breast, cervix uteri, corpus uteri, ovary, bladder, head and neck, endometrial, osteosarcoma, prostate, and neuroblastoma.

5. A compound with the structure

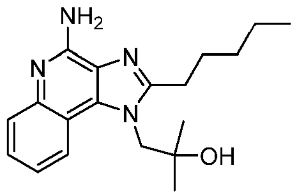

or a pharmaceutically acceptable salt thereof or a pharmaceutical composition as claimed in claim 1, for use in treating a disease or a condition wherein activation of TLR7 and/or TLR8 provides a benefit in a subject in need thereof.

**Patentansprüche**

1. Pharmazeutische Zusammensetzung, umfassend:

   1-(4-Amino-2-pentyl-1*H*-imidazo[4,5-*c*]chinolin-1-yl)-2-methylpropan-2-ol oder ein pharmazeutisch unbedenkliches Salz davon; und
   einen pharmazeutisch unbedenklichen Träger und/oder ein Adjuvans.

2. Pharmazeutische Zusammensetzung nach Anspruch 1 zur Verwendung bei der Behandlung einer Virusinfektion, von Krebs, einer allergischen Erkrankung oder einer Lebererkrankung bei einem Subjekt, das dessen bedarf.

3. Verbindung mit der Struktur

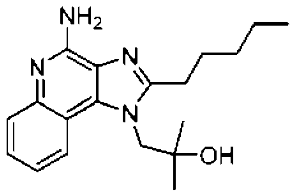

oder pharmazeutisch unbedenkliches Salz davon zur Verwendung bei der Behandlung einer Virusinfektion, von Krebs, einer allergischen Erkrankung oder einer Lebererkrankung bei einem Subjekt, das dessen bedarf.

4. Verbindung oder pharmazeutisch unbedenkliches Salz davon nach Anspruch 3 oder pharmazeutische Zusammensetzung zur Verwendung nach Anspruch 2, wobei der Krebs ausgewählt ist aus der Gruppe bestehend aus Speiseröhrenkrebs, Magenkrebs, Kolonkrebs, Rektalkrebs, Bauchspeicheldrüsenkrebs, Lungenkrebs, Brustkrebs, Zervixkrebs, Uteruskrebs, Eierstockkrebs, Blasenkrebs, Kopf- und Halskrebs, Endometriumkrebs, Osteosarkom, Prostatakrebs und Neuroblastom.

5. Verbindung mit der Struktur

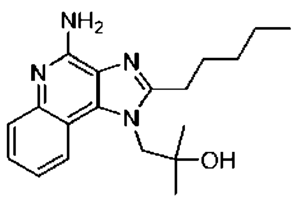

oder pharmazeutisch unbedenkliches Salz davon oder pharmazeutische Zusammensetzung nach Anspruch 1 zur Verwendung bei der Behandlung einer Erkrankung oder eines Zustands, wobei eine Aktivierung von TLR7 und/oder TLR8 einen Nutzen bereitstellt, bei einem Subjekt, das dessen bedarf.

**Revendications**

1. Composition pharmaceutique comprenant :

   1-(4-amino-2-pentyl-1*H*-imidazo[4,5-c]quinolin-1-yl)-2-méthylpropan-2-ol ou un sel acceptable pharmaceutiquement de celui-ci ; et
   un support acceptable pharmaceutiquement et/ou un adjuvant.

2. Composition pharmaceutique selon la revendication 1, destinée à être utilisée dans le traitement d'une infection virale, d'un cancer, d'une maladie allergique ou d'une maladie hépatique chez un sujet qui en a besoin.

3. Composé présentant la structure

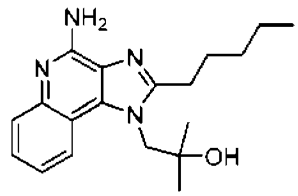

ou un sel acceptable pharmaceutiquement de celui-ci destiné à être utilisé dans le traitement d'une infection virale, d'un cancer, d'une maladie allergique ou d'une maladie hépatique chez un sujet qui en a besoin.

4. Composé ou sel acceptable pharmaceutiquement de celui-ci selon la revendication 3, ou composition pharmaceutique destiné(e) à être utilisé(e) selon la revendication 2, ledit cancer étant choisi dans le groupe constitué de l'œsophage, de l'estomac, du côlon, du rectum, du pancréas, du poumon, du sein, du col de l'utérus, du corps utérin, de l'ovaire, de la vessie, de la tête et du cou, de l'endomètre, de l'ostéosarcome, de la prostate et du neuroblastome.

5. Composé présentant la structure

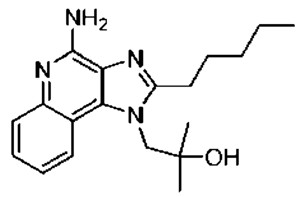

ou sel acceptable pharmaceutiquement de celui-ci ou composition pharmaceutique selon la revendication 1, destiné(e) à être utilisé(e) dans le traitement d'une maladie ou d'une affection dans laquelle l'activation de TLR7 et/ou TLR8 procure un avantage chez un sujet qui en a besoin.

Fig. 1A

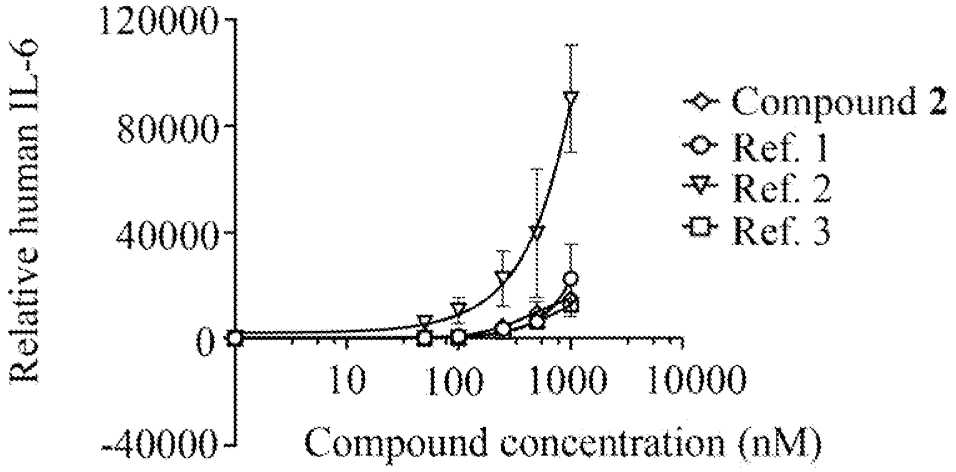


Fig. 1B

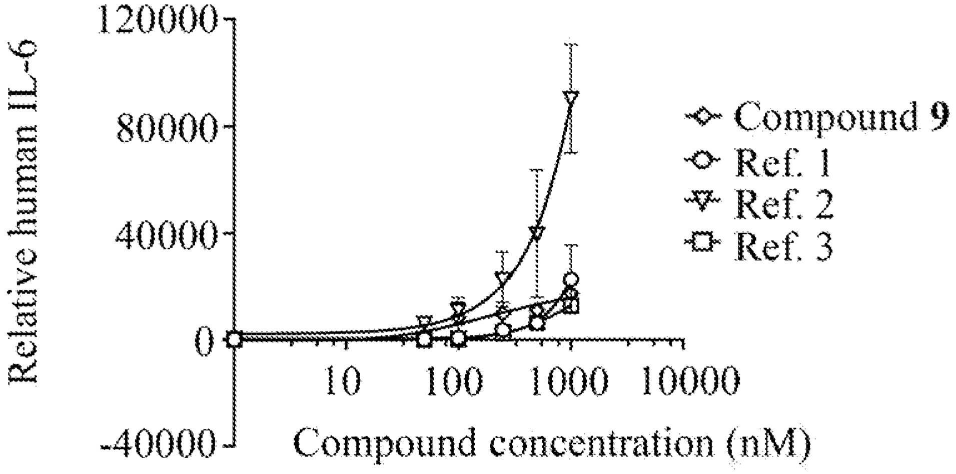


Fig. 1C

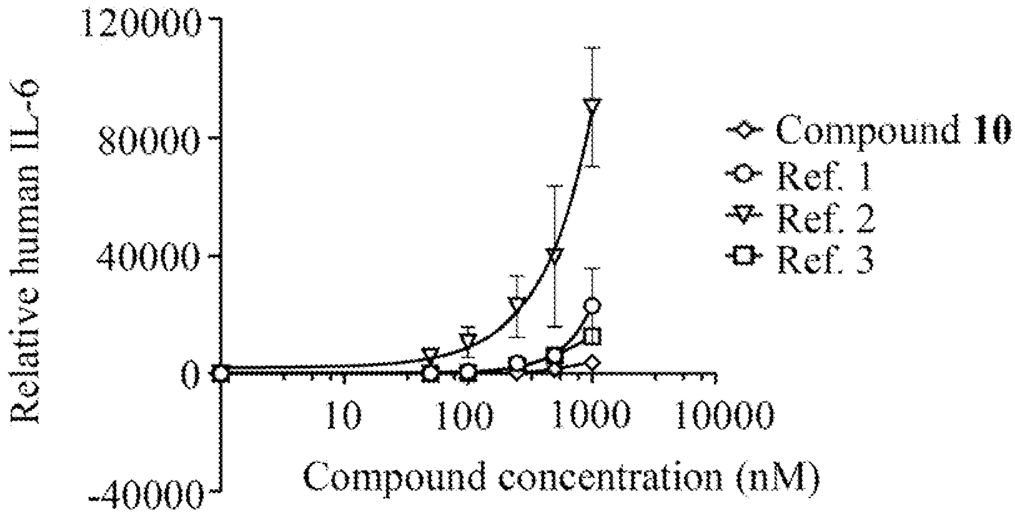

Fig. 2A

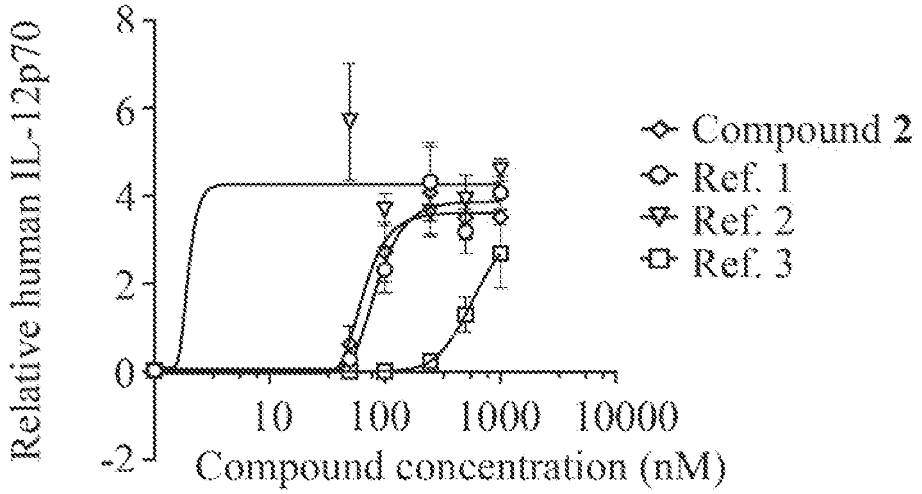


Fig. 2B

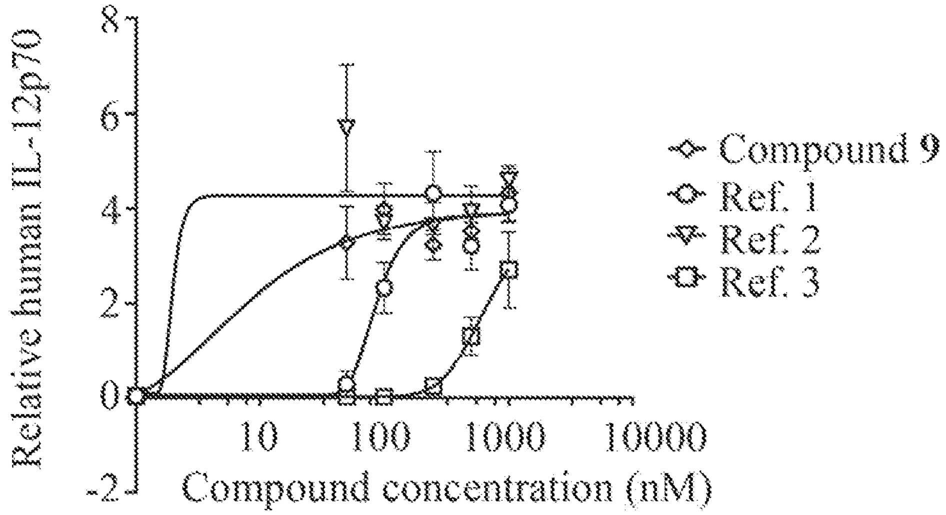


Fig. 2C

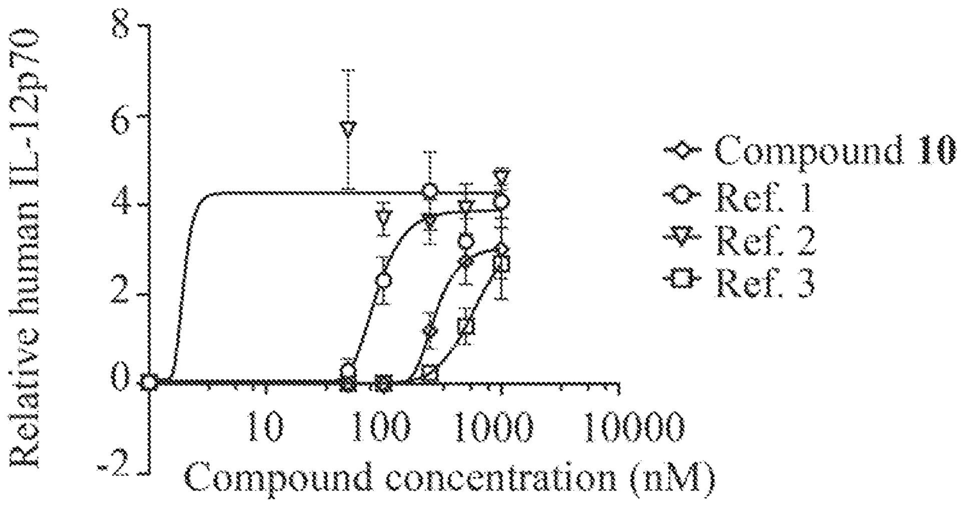

Fig. 3A

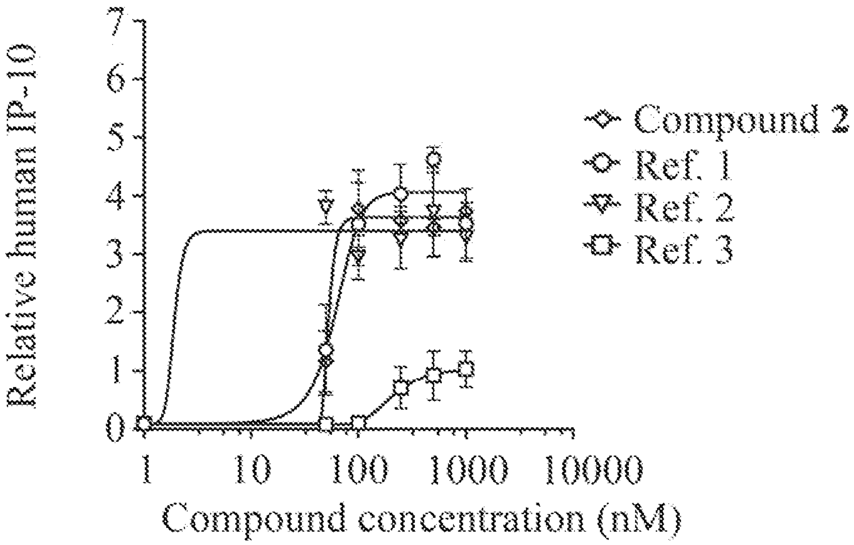


Fig. 3B

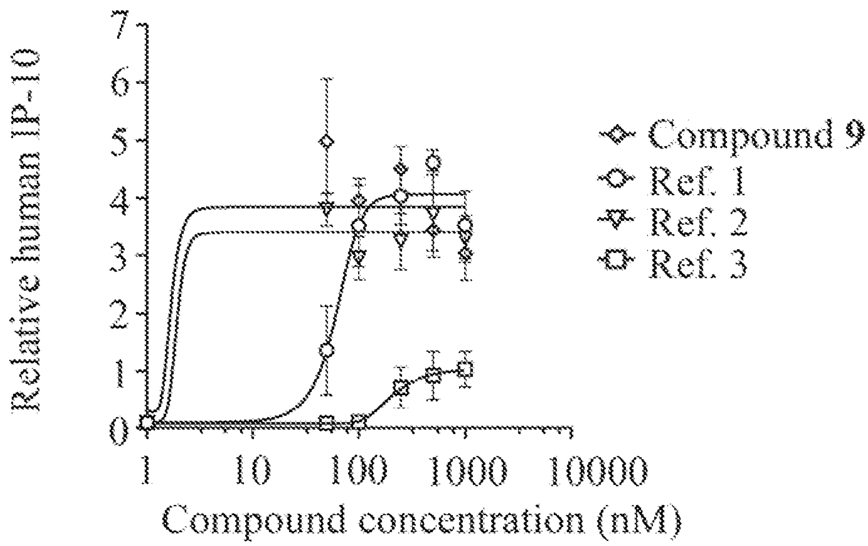


Fig. 3C

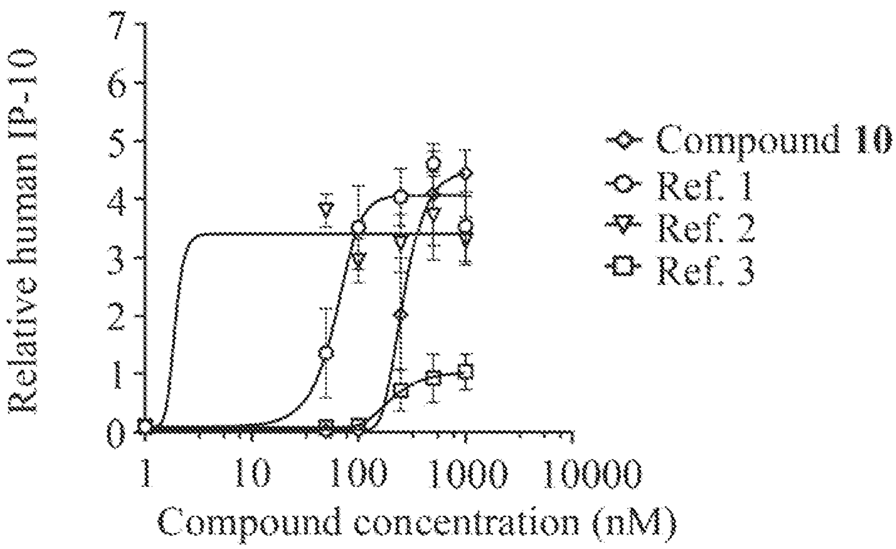

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8728486 B
- US 9334268 B
- US 9884866 B
- US 20190062329 A
- WO 2015162075 A1
- WO 2020160054 A1
- WO 2006028962 A2
- WO 2012167081 A1
- WO 2020252015 A
- WO 2019048036 A1
- WO 2004058759 A1
- WO 2002046189 A2
- WO 2014201245 A1


### Non-patent literature cited in the description


- **CE SHI et al.** *ACS Medicinal Chemistry Letters*, June 2012, vol. 3 (6), 501-504
- **IRA MUSMUCA et al.** *J. Chem. Inf. Model*, 2009, vol. 49, 1777-1786
- **SMITH ALYSON J et al.** *Vaccine*, 09 July 2016, vol. 34 (36), 4304-4312
- U.S. Department of Health and Human Services Food and Drug Administration discloses. U.S. Department of Health and Human Services Food and Drug Administration
- **GANAPATHI L et al.** *PLOS ONE*, 2015, vol. 10 (8), 0134640